(19) Europäisches Patentamt
European Patent Office
Office européen des brevets

(11) Publication number: **0 125 714**
**B1**

(12) # EUROPEAN PATENT SPECIFICATION

(45) Date of publication of patent specification: **11.11.87**

(51) Int. Cl.[4]: **C 07 D 205/04** // C07C91/06

(21) Application number: **84200534.0**

(22) Date of filing: **13.04.84**

(54) **Preparation of 1-substituted azetidin-3-ol and acid halid thereof.**

(30) Priority: **04.05.83 GB 8312104**

(43) Date of publication of application:
**21.11.84 Bulletin 84/47**

(45) Publication of the grant of the patent:
**11.11.87 Bulletin 87/46**

(84) Designated Contracting States:
**AT BE CH DE FR GB IT LI LU NL SE**

(56) References cited:
**GB-A-1 236 078**

**CHEMICAL COMMUNICATIONS, no. 1, 10th January 1968, page 93; S.S. CHATTERJEE et al.: "Synthesis of azetidin-3-ol"**

**TETRAHEDRON LETTERS, no. 39, 1966, pages 4691-4694, Pergamon Press Ltd., GB; V.R. GAERTNER: "Cyclization of 1-alkylamino-3-halo-2-alkanols to 1-alkyl-3-azetidinols"**

**THE JOURNAL OF ORGANIC CHEMISTRY, vol. 37, no. 24, 1st December 1972, pages 3953-3955, American Chemical Society; A.G. ANDERSON Jr. et al.: "The synthesis of azetidine-3-carboxylic acid"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Orr, Alexander Ferguson**
**6 Amber Close**
**Teynham - nr. Sittingbourne Kent (GB)**

(74) Representative: **Hunter, Keith Roger Ian et al**
**4 York Road**
**London SE1 7NA (GB)**

**EP 0 125 714 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of 1-benzyl azetidin-3-ol and its acid halide, which compounds are intermediates for the preparation of biologically active compounds.

It is known, for example, from J. Org. Chem. *37*, 3953 (1972) that 1-benzhydrylazetidin-3-ol may be prepared by the reaction of benzhydrylamine and epichlorohydrin in methanol. However, the introduction of a benzhydryl group is very inconvenient for an economically practicable synthesis route, since the size of that group greatly increases the bulk of material to be processed, only to be removed once its protective function is no longer required. It would be economically very desirable to use a protective group less bulky than the benzhydryl group, but previous attempts to react epichlorohydrin with benzylamine have failed to produce any significant yield of the desired cyclised azetidine product, as is described in Chemical Communications, 1/1968, p.93.

Applicants have surprisingly found that the use of an aqueous reaction medium enables useful yields of 1-benzyl azetidines to be obtained from an epoxy halide and benzylamine. Accordingly the invention provides a process for the preparation of 1-benzyl azetidine-3-ol or an acid halide thereof, which comprises the following steps:

(a) reacting an epoxy halide of formula:

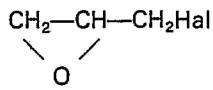

I

in which Hal represents a halogen atom with benzylamine to form an addition product of formula:

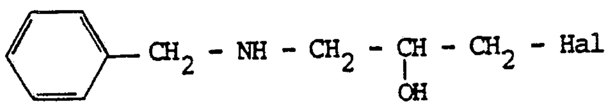

II

(b) cyclising said addition product in an aqueous medium comprising at least 25% water by volume, to form the acid halide of 1-benzyl azetidin-3-ol, and

(c) when 1-benzyl azetidin-3-ol itself is required, treating said acid halide with a base.

Step (a), the reaction of the epoxy halide of formula I with benzylamine, is preferably carried out by mixing the reactants in an organic solvent, for example a hydrocarbon solvent such as cyclohexane. Suitable reaction temperatures are from 10° to 50°C and suitable reaction times are from 12 to 36 hours. The addition product of formula II may be recovered from the reaction mixture by conventional procedures and, if desired, may be purified, for example by recrystallisation, before step (b).

The cyclization of addition product of formula II is carried out in an aqueous medium which may be water itself or a mixture of water and an organic solvent, the proportion of water in the mixture being at least 25% by volume. The organic solvent may be chosen from a wide range of organic compounds, for example hydrocarbons, such as toluene, halo-hydrocarbons such as carbon tetrachloride, tetrachloroethane or bromobenzene alcohols such as methanol, isopropanol or n-butanol, ketones such as methyl isobutyl ketone, cyclo-ethers such as dioxan, esters such as ethyl acetate or nitro-compounds such as nitrobenzene. The proportion of water in the mixture is suitably from 25 to 75% by volume. The cyclisation of step (b) is preferably carried out at elevated temperature for example from 50° to 150°C. Conveniently the cyclization is carried out at the boiling point of the aqueous medium under reflux.

The immediate product of the cyclisation step (b) is the acid halide of 1-benzyl azetidin-3-ol. Treatment of this acid halide in step (c) with a base, for example an alkali metal hydroxide, gives 1-benzyl azetidin-3-ol itself, which may be recovered and purified by conventional procedures.

As mentioned above, 1-benzyl azetidin-3-ol and its acid halide are useful intermediates. Thus, they may be converted by known procedures, for example, via the corresponding 3-cyanoazetidine derivative, to azetidine-3-carboxylic acid derivatives, which exhibit plant growth regulant properties, especially the property of rendering sterile the male parts of plants.

The invention therefore includes the use of 1-benzyl azetidin-3-ol or its acid halide, prepared by the process of the invention, as intermediates for the preparation of azetidine-3-carboxylic acid derivatives.

The invention is illustrated in the following Examples.

Example 1

Preparation of 1-benzylazetidin-3-ol

(a) Epichlorohydrin (46.25 g) and benzylamine (53.5 g) in cyclohexane (250 ml) were stirred together at ambient temperature for 24 hours. The precipitate formed was filtered off and recrystallized from toluene to give N-benzyl-3-amino-1-chloropropan-2-ol as a crystalline solid, m.p. 70—71°C, in 55% yield.

(b) N-benzyl-3-amino-1-chloropropan-2-ol (10 g, prepared as in (a)) was heated under reflux in water (150 ml) with stirring for 24 hours. The reaction mixture was cooled, and the aqueous solution was decanted from insoluble material and made alkaline by the addition of solid sodium hydroxide. The oil which separated was extracted with dichloromethane and the extracts were washed with water and dried.

The solvent was then removed under reduced pressure and the residue was purified by chromatography on silica gel using acetone as eluent, followed by recrystallization from toluene to give 1-benzylazetidin-3-ol as a crystalline solid, m.p. 64.5—65.5°C, in 27% yield.

Analysis Calculated for $C_{10}H_{13}NO$: C 73.6%; H 8.0%; N 8.6%
Found C 73.5%; H 8.3%; N 8.6%
NMR ($CDCl_3$) 2.95 (m, 2H); 3.60 (m, 4H), 4.40 (m, 1H); 7.30 (m, 5H); 3.30 (1H, broad, OH)

Examples 2 to 12

The procedure of Example 1 was repeated, except that in step (b) the water was replaced by a 1:1 mixture by volume of water and an organic solvent. The results of these Examples are summarized in Table I.

Comparative Experiments

The procedure of Example 1 was repeated, except that in step (b) the water was replaced in turn by ethanediol, acetonitrile, methanol and butanol. In each case no 1-benzylazetidin-3-ol was formed.

TABLE I

| Example | organic solvent component | yield of 1-benzylazetidin-3-ol % |
|---|---|---|
| 2 | toluene | 28 |
| 3 | tetrachloroethane | 27 |
| 4 | n-butanol | 26 |
| 5 | bromobenzene | 25 |
| 6 | methanol | 26 |
| 7 | nitrobenzene | 25 |
| 8 | iso-propanol | 26 |
| 9 | methyl isobutyl ketone | 26 |
| 10 | dioxane | 27 |
| 11 | carbon tetrachloride | 27 |
| 12 | ethyl acetate | 25 |

Example 13

Use of 1-benzylazetidin-3-ol to prepare azetidin-3-carboxylic acid

(a) 1-Benzylazetidin-3-ol (5.0 g, prepared as in Example 1), methane sulphonyl chloride (3.52 g) and triethylamine (6 ml) in dichloromethane (40 ml) were stirred together for 18 hours. The mixture was filtered and the solvent was removed under reduced pressure. The residue was purified by chromatography on silica gel using isopropanol in dichlormethane as eluent to give the mesylate of 1-benzylazetidin-3-ol, yield 4.25 g.

(b) The mesylate of 1-benzylazetidin-3-ol (1.7 g, prepared as in (a)), and sodium cyanide (1.2 g) were stirred together in water (1 ml) and dimethylformamide (20 ml) at 60°C for 16 hours. The solvent was removed under reduced pressure and the residue was purified by chromatography on silica gel using isopropanol in dichloromethane as eluent to give 1-benzyl-3-cyanoazetidine, yield 0.5 g.

(c) 1-Benzyl-3-cyanoazetidine (0.5 g, prepared as in (b)) in saturated barium hydroxide solution (10 ml) was heated under reflux for 30 hours. The reaction mixture was cooled, saturated with gaseous carbon dioxide and filtered. The solvent was removed from the filtrate under reduced pressure to give 1-benzylazetidine-3-carboxylic acid in 80% yield.

(d) 1-Benzylazetidine-3-carboxylic acid (0.5 g, prepared as in (c)) in methanol (15 ml) was hydrogenated in the presence of a 5% palladium on carbon catalyst at room temperature. The catalyst was filtered off and the solvent removed from the filtrate under reduced pressure to give azetidin-3-carboxylic acid in 90% yield.

**Claims**

1. A process for the preparation of 1-benzyl azetidin-3-ol or an acid halide thereof, which comprises the following steps:

(a) reacting an epoxy halide of formula:

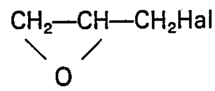

I

in which Hal represents a halogen atom with benzylamine to form an addition product of formula:

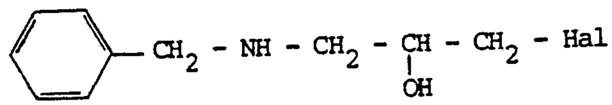

II

(b) cyclizing said addition product in an aqueous medium comprising at least 25% water by volume, to form the acid halide of 1-benzyl azetidin-3-ol, and

(c) when 1-benzyl azetidin-3-ol itself is required, treating said acid halide with a base.

2. A process as claimed in claim 1, in which the aqueous medium in step (b) is either water or a mixture of water and an organic solvent selected from a hydrocarbon, halohydrocarbon, alcohol, ketone, cycloether, ester or nitro-compound.

3. A process as claimed in claim 2 in which the proportion of water in the mixture is from 25 to 75% by volume.

4. A process as claimed in any preceding claim in which the cyclization of step (b) is carried out at a temperature from 50° to 150°C.

5. A process as claimed in claim 4 in which the cyclization of step (b) is carried out under reflux at the boiling point of the aqueous medium.

6. A process as claimed in any preceding claim in which the treatment of step (c) is carried out with an alkali metal hydroxide.

7. The use of 1-benzyl azetidin-3-ol or its acid halide, prepared by a process as claimed in any preceding claim, as intermediates for the preparation of azetidine-3-carboxylic acid derivatives.

**Patentansprüche**

1. Verfahren zur Herstellung von 1-Benzylazetidin-3-ol oder eines Säurehalogenids hievon, welches Verfahren die folgenden Stufen umfasst:

(a) Umsetzen eines Epoxyhalogenids der Formel:

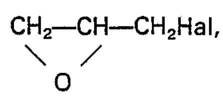

(I)

worin Hal ein Halogenatom bedeutet, mit Benzylamin zur Ausbildung eines Additionsproduktes der Formel:

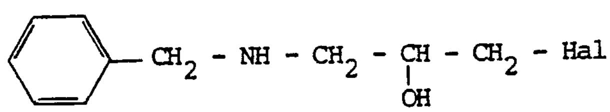

II

(b) Cyclisieren dieses Reaktionsproduktes in einem wenigstens 25 Volums-% Wasser umfassenden wäßrigen Medium zur Ausbildung des Säurehalogenids von 1-Benzylazetidin-3-ol; und

(c) falls 1-Benzylazetidin-3-ol selbst benötigt wird, Behandeln dieses Säurehalogenids mit einer Base.

2. Verfahren nach Anspruch 1, worin das wäßrige Medium in Stufe (b) entweder Wasser oder ein Gemisch aus Wasser mit einem organischen Lösungsmittel, ausgewählt unter einem Kohlenwasserstoff, Halogenkohlenwasserstoff, Alkohol, Keton, cyclischem Ether, Ester oder einer Nitroverbindung, ist.

3. Verfahren nach Anspruch 2, worin der Wasseranteil in dem Gemisch von 25 bis 75 Volums-% beträgt.

4. Verfahren nach einem der vorstehenden Ansprüche, worin die Cyclisierung in Stufe (b) bei einer Temperatur von 50 bis 150°C ausgeführt wird.

5. Verfahren nach Anspruch 4, worin die Cyclisierung in Stufe (b) unter Rückfluß beim Siedepunkt des wäßrigen Mediums ausgeführt wird.

6. Verfahren nach einem der vorstehenden Ansprüche, worin die Behandlung in Stufe (c) mit einem Alkalimetallhydroxid ausgeführt wird.

7. Verwendung von 1-Benzylazetidin-3-ol oder seinem Säurehalogenid, hergestellt nach einem

Verfahren gemäß einem der vorstehenden Ansprüche, als Zwischenprodukte für die Herstellung von Azetidin-3-crbonsäure-Derivaten.

**Revendications**

1. Un procédé pour la préparation du 1-benzylazétidin-3-ol ou d'un halogénure d'acide correspondant, qui comprend les étapes suivantes:

(a) réaction d'un époxy halogénure de formule:

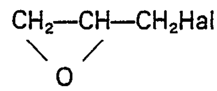

I

où Hal représente un atome d'halogène avec la benzylamine de manière à former un produit d'addition de formule:

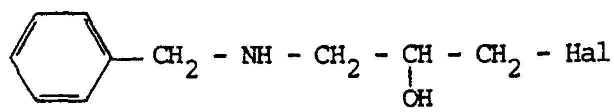

II

(b) cyclisation de ce produit d'addition dans un milieu aqueux comprenant au moins 25% en volume d'eau, pour former l'halogénure d'acide du 1-benzyl azétidin-3-ol et

(c) quand le benzyl azétidin-3-ol lui-même est nécessaire, traitement de cet halogénure d'acide par une base.

2. Un procédé selon la revendication 1, dans lequel le milieu aqueux dans l'étape (b) est de l'eau ou un mélange d'eau et d'un solvant organique choisi parmi un hydrocarbure, un halohydrocarbure, un alcool, une cétone, un cyclo-éther ou un composé nitré.

3. Un procédé selon la revendication 2, dans lequel la proportion d'eau dans le mélange est de 25 à 75% en volume.

4. Un procédé selon l'une quelconque des revendications précédentes, dans lequel la cyclisation de l'étape (b) est effectuée à une température de 50° à 150°C.

5. Un procédé selon la revendication 4, dans lequel la cyclisation de l'étape (b) est effectuée au reflux au point d'ébullition du milieu aqueux.

6. Un procédé selon l'une quelconque des revendications précédentes, dans lequel le traitement de l'étape (c) est effectué avec un hydroxyde de métal alcalin.

7. L'utilisation du 1-benzyl azétidin-3-ol ou d'un halogénure d'acide correspondant, préparé par un procédé selon une quelconque des revendications précédentes, comme produits intermédiaires pour la préparation de dérivés d'acide azétidine-3-carboxylique.